(19) Europäisches Patentamt / European Patent Office / Office européen des brevets

(11) **EP 4 122 461 A1**

(12) **EUROPEAN PATENT APPLICATION**

(43) Date of publication:
**25.01.2023 Bulletin 2023/04**

(21) Application number: **21187449.0**

(22) Date of filing: **23.07.2021**

(51) International Patent Classification (IPC):
**A61K 31/245** (2006.01)     **A61K 31/4425** (2006.01)
**A61P 31/14** (2006.01)

(52) Cooperative Patent Classification (CPC):
(C-Sets available)
**A61P 31/14; A61K 31/245; A61K 31/4425**    (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Freie Universität Berlin**
  **14195 Berlin (DE)**
• **Deutsches Primatenzentrum GmbH**
  **Leibniz-Institut**
  **für Primatenforschung**
  **37077 Göttingen (DE)**

(72) Inventors:
• **HEMPEL, Tim**
  **10589 Berlin (DE)**

• **ELEZ, Katarina**
  **12163 Berlin (DE)**
• **RAICH, Lluís**
  **10439 Berlin (DE)**
• **NOÉ, Frank**
  **10825 Berlin (DE)**
• **KRÜGER, Nadine**
  **37075 Göttingen (DE)**
• **HOFFMANN, Markus**
  **37075 Göttingen (DE)**
• **PÖHLMANN, Stefan**
  **37073 Göttingen (DE)**

(74) Representative: **Maikowski & Ninnemann**
**Patentanwälte Partnerschaft mbB**
**Postfach 15 09 20**
**10671 Berlin (DE)**

(54) **PHARMACEUTICAL COMPOSITION FOR TREATING COVID-19 COMPRISING OTAMIXABAN AND AT LEAST ONE OF CAMOSTAT AND NAFAMOSTAT**

(57)    The invention relates to a pharmaceutical composition that is appropriate to treat or prevent SARS-CoV-2-related diseases such as COVID-19. The pharmaceutical composition comprises a pharmaceutically effective amount of i) otamixaban or a pharmaceutically acceptable salt thereof and ii) at least one of camostat, a pharmaceutically acceptable salt of camostat, nafamostat, and a pharmaceutically acceptable salt of nafamostat.

FIG 1B

EP 4 122 461 A1

(52) Cooperative Patent Classification (CPC): (Cont.)

C-Sets
**A61K 31/245, A61K 2300/00;**
**A61K 31/4425, A61K 2300/00**

**Description**

The present invention relates to a pharmaceutical composition according to the preamble of claim 1, to further medical uses of such a pharmaceutical composition according to the preambles of claims 7 and 8 as well as to the further medical uses of the active ingredients of such a pharmaceutical composition according to the preambles of claims 9 and 10.

Severe acute respiratory syndrome coronavirus 2 (SARS-CoV-2) infection causes coronavirus disease 2019 (COVID-19), a potentially fatal disease. The first cases of this illness were reported in late December 2019 in Wuhan, China. Due to the high human-to-human transmission rate of SARS-CoV-2, the World Health Organization declared the outbreak a pandemic [1]. By early March 2021, the virus has infected more than 116.1 million and killed more than 2.5 million people worldwide [2]. With no robust therapeutic options available at present, the development of new antivirals against SARS-CoV-2 is of imminent importance.

SARS-CoV-2 exploits two membrane-bound host proteins for cell entry - angiotensin-converting enzyme 2 (ACE2) and transmembrane serine protease 2 (TMPRSS2) [4]. Specifically, the spike (S) protein of SARS-CoV-2 binds to ACE2 and is subsequently cleaved by the transmembrane serine protease TMPRSS2, allowing membrane fusion and cell entry.

The low risk of SARS-CoV-2 infection in groups with decreased levels of TMPRSS2, such as infants/children [5] and androgen-deprived prostate cancer patients [6], is consistent with the suggested SARS-CoV-2 dependence on TMPRSS2. The S protein depends on TMPRSS2 for priming (proteolytic separation of its subunits) in human airway cells (primary targets of the virus) [4, 7], even though it also relies on the endosomal cysteine protease cathepsin L for entry into some cell lines [4, 8]. Additionally, recent evidence suggests that coagulation factors such as factor Xa (fXa) can directly cleave S and facilitate SARS-CoV-2 cell entry [9].

TMPRSS2 also activates other coronaviruses [10-16], as well as several strains of the influenza A virus [17-19]. TMPRSS2 is dispensable for normal development and homeostasis in mice [20], suggesting that blockade of this protease might not be associated with substantial unwanted side effects.

Two synthetic serine protease inhibitors, camostat and nafamostat, approved in Japan for treatment of pancreatitis, inhibit TMPRSS2 and block SARS-CoV-2 infection [4, 20, 21]. Beside these small molecules, protein-based inhibitors such as Alpha-1-antitrypsin [23] and Aprotinin (BPTI) [24], have been proposed as potential treatment options. However, demand for potent and easily administrable TMPRSS2 inhibitors still exists.

Otamixaban is a potent non-covalent synthetic inhibitor of factor Xa [25], investigated as an anticoagulant for managing acute coronary syndrome. It entered phase III clinical trials but was deemed inferior to unfractionated heparin combined with eptifibatide for reducing ischemic events [26].

Rensi et al. [27] identified otamixaban as a potential TMPRSS2 inhibitor in their virtual screening. The inventors corroborated this hit computationally by docking the compound against several protein conformations extracted from extensive molecular dynamics simulations [3]. Otamixaban was experimentally identified by the screening pipelines of both, the NIH/NCATS Cheminformatics group led by Shen [28] and the Noé lab, using the NIH/NCATS TMPRSS2 high throughput screening (HTS) assay [29].

It is an object of the present invention to provide pharmaceutical compositions that are more appropriate for treating or preventing SARS-CoV-2-related infections than pharmaceutical compositions known from prior art.

This object is achieved with a pharmaceutical composition comprising a pharmaceutically effective amount of i) otamixaban or a pharmaceutically acceptable salt thereof and ii) at least one of camostat, a pharmaceutically acceptable salt of camostat, nafamostat, and a pharmaceutically acceptable salt of nafamostat.

Based on TMPRSS2 enzyme activity assay results, SARS-CoV-2-S-driven cell entry assays in a human lung cell line (Calu-3), and precision cut human lung slices (PCLS) as well as 109-$\mu$s *in silico* molecular dynamics simulations, the inventors found, under *in vitro* conditions, otamixaban to be a weak inhibitor of TMPRSS2 with an IC50 value that is two to three orders of magnitude above that of more established TMPRSS2 inhibitors camostat and nafamostat.

Surprisingly, the inventors observed strong synergistic effects when otamixaban is combined with camostat or nafamostat. Therefore, a combination preparation of otamixaban with camostat or nafamostat appears to be a very potent drug for a therapy against COVID-19.

In an embodiment, a (molar) ratio between i) the pharmaceutically effective amount of camostat, a pharmaceutically acceptable salt of camostat, nafamostat, or a pharmaceutically acceptable salt of camostat and ii) the pharmaceutically effective amount of otamixaban or a pharmaceutically acceptable salt of otamixaban lies in a range of from 1:10000 to 1:1000, in particular of from 1:8000 to 1:2000, in particular of from 1:7000 to 1:3000, in particular of from 1:6000 to 1: 4000, in particular of from 1:5000 to 1:4500. Thus, a comparatively small amount of camostat or nafamostat (or a pharmaceutically acceptable salt thereof) is already sufficient to induce the observed synergistic effect when combined with otamixaban. To be more precise, it is sufficient if camostat or nafamostat (or a pharmaceutically acceptable salt thereof) are present in the nanomolar or subnanomolar range in a solution comprising otamixaban to induce the observed synergistic effect.

In an embodiment, the pharmaceutical composition comprises a pharmaceutically effective amount of i) otamixaban or a pharmaceutically acceptable salt thereof and ii) camostat or a pharmaceutically acceptable salt of camostat.

In an embodiment, the pharmaceutical composition comprises a pharmaceutically effective amount of i) otamixaban or a pharmaceutically acceptable salt thereof and ii) camostat.

In an embodiment, the pharmaceutical composition comprises a pharmaceutically effective amount of otamixaban and camostat.

In an embodiment, the pharmaceutical composition comprises a pharmaceutically effective amount of a pharmaceutically acceptable salt of otamixaban and camostat.

In an embodiment, the pharmaceutical composition comprises a pharmaceutically effective amount of a pharmaceutically acceptable salt of otamixaban and a pharmaceutically acceptable salt of camostat.

In an embodiment, the pharmaceutical composition comprises a pharmaceutically effective amount of otamixaban and a pharmaceutically acceptable salt of camostat.

In an embodiment, the pharmaceutical composition comprises a pharmaceutically effective amount of i) otamixaban or a pharmaceutically acceptable salt thereof and ii) nafamostat or a pharmaceutically acceptable salt of nafamostat.

In an embodiment, the pharmaceutical composition comprises a pharmaceutically effective amount of i) otamixaban or a pharmaceutically acceptable salt thereof and ii) nafamostat.

In an embodiment, the pharmaceutical composition comprises a pharmaceutically effective amount of otamixaban and nafamostat.

In an embodiment, the pharmaceutical composition comprises a pharmaceutically effective amount of a pharmaceutically acceptable salt of otamixaban and nafamostat.

In an embodiment, the pharmaceutical composition comprises a pharmaceutically effective amount of a pharmaceutically acceptable salt of otamixaban and a pharmaceutically acceptable salt of nafamostat.

In an embodiment, the pharmaceutical composition comprises a pharmaceutically effective amount of otamixaban and a pharmaceutically acceptable salt of nafamostat.

In an embodiment, the pharmaceutically active ingredients of the before-mentioned combinations are the only pharmaceutically active ingredients in the pharmaceutical composition.

In an aspect, the present invention relates to the further medical use of a pharmaceutical composition according to the preceding explanations. This further medical use is directed to a treatment or prevention of an infection with severe acute respiratory syndrome coronavirus 2 (SARS-CoV-2).

In an aspect, the further medical use of the pharmaceutical composition is directed to a treatment or prevention of COVID-19.

In an aspect, the present invention relates to the further medical use of the pharmaceutically active ingredients of the pharmaceutical composition according to the preceding explanations. According to this aspect, a combination of i) otamixaban or a pharmaceutically acceptable salt thereof and ii) at least one of camostat, a pharmaceutically acceptable salt of camostat, nafamostat, and a pharmaceutically acceptable salt of nafamostat is used for treating or preventing an infection with severe acute respiratory syndrome coronavirus 2 (SARS-CoV-2).

In an aspect, the further medical use of such a substance combination is directed to a treatment or prevention of COVID-19.

In an aspect, the present invention relates to a medical method of treating or preventing an infection with severe acute respiratory syndrome coronavirus 2 (SARS-CoV-2) by administering a pharmaceutical composition according to the preceding explanations to a patient in need thereof.

In an aspect, the present invention relates to a medical method of treating or preventing COVID-19 by administering a pharmaceutical composition according to the preceding explanations to a patient in need thereof.

In an aspect, the present invention relates to a medical method of treating or preventing an infection with severe acute respiratory syndrome coronavirus 2 (SARS-CoV-2) by administering a combination of i) otamixaban or a pharmaceutically acceptable salt thereof and ii) at least one of camostat, a pharmaceutically acceptable salt of camostat, nafamostat, and a pharmaceutically acceptable salt of nafamostat to a patient in need thereof. In this context, the before-mentioned pharmaceutically active ingredients (i.e., otamixaban, camostat and nafamostat or the pharmaceutically acceptable salts thereof) are administered in a pharmaceutically active amount.

In an aspect, the present invention relates to a medical method of treating or preventing COVID-19 by administering a combination of i) otamixaban or a pharmaceutically acceptable salt thereof and ii) at least one of camostat, a pharmaceutically acceptable salt of camostat, nafamostat, and a pharmaceutically acceptable salt of nafamostat to a patient in need thereof. Also in this context, the before-mentioned pharmaceutically active ingredients are administered in a pharmaceutically active amount.

In an embodiment, the pharmaceutically active ingredients are administered orally or by an intravenous, intramuscular or intraperitoneal injection.

All embodiments of the pharmaceutical composition can be combined in any desired way and can be transferred either individually or in any arbitrary combination to the described further medical uses, to the described substance combination, and to the described methods. All embodiments of the described further medical uses can be combined in any desired way and can be transferred either individually or in any arbitrary combination to the described pharmaceutical composition, the described substance combination, and to the described methods. All embodiments of the substance combination can be combined in any desired way and can be transferred either individually or in any arbitrary combination to the described pharmaceutical composition, to the described medical uses, and to the described methods. Finally, all embodiments of the methods can be combined in any desired way and can be transferred either individually or in any arbitrary combination to the described pharmaceutical composition, to the described further medical uses, and to the described substance combination.

Further details of aspects of the present invention will be explained in the following making reference to exemplary embodiments and accompanying Figures. In the Figures:

Figure 1A    shows the chemical structure of otamixaban at physiological pH;

Figure 1B    shows the dose-response curves in Calu-3 cells with their respective IC50 estimates;

Figure 1C    shows the Inhibition of SARS-CoV-2 infection of precision cut human lung slices (PCLS) by otamixaban, camostat and nafamostat;

Figure 2A    shows the antiviral activity of otamixaban in combination with 1 nM camostat or 0.1 nM nafamostat compared with otamixaban alone; and

Figure 2B    shows the combination index as a function of inhibition strength.

## Experimental Results

Otamixaban was identified as a potential TMPRSS2 inhibitor in the NIH/NCATS TMPRSS2 high throughput screening (HTS) assay [29]. This is a single replicate experiment conducted at room temperature which is scalable to large amounts of different drug candidates (1536-well plates). The HTS assay indicated that otamixaban shows inhibitory activity against TMPRSS2 (data not shown). Subsequently, activity confirmation was performed in a different facility (cf. Materials and Methods section below) at physiological temperature with multiple replicates, using a similar, although low throughput, TMPRSS2 activity assay. The inventors found that, as expected, both assays yield comparable results (data not shown).

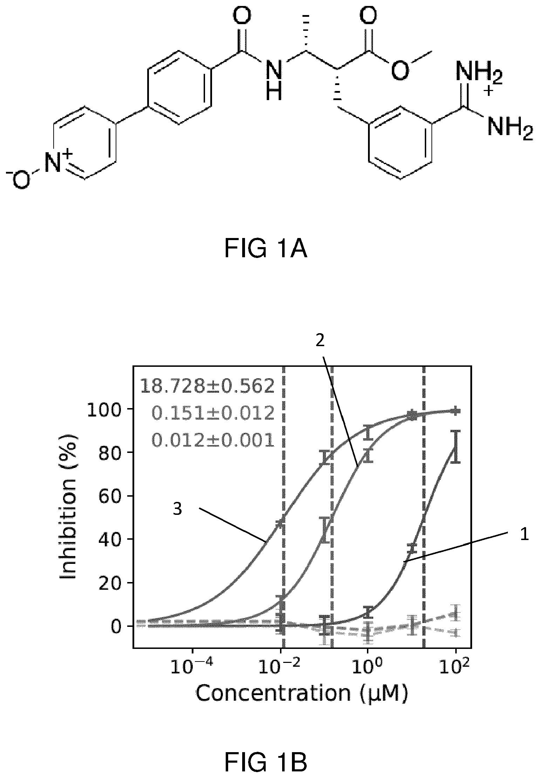

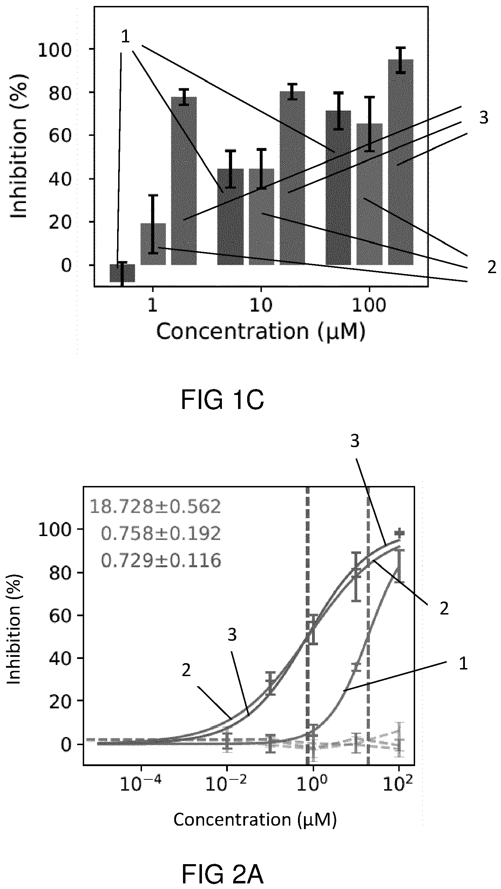

The inventors next tested otamixaban in the Calu-3 cell line where they found it to inhibit SARS-CoV-2 S-mediated viral entry, but not entry driven by vesicular stomatitis virus (VSV) glycoprotein (VSV-G), which does not require TMPRSS2 activity to mediate cell entry. Inhibition of SARS-2-S-driven cell entry by otamixaban was much less potent compared to camostat and nafamostat (Fig. 1B). Being in the lower $\mu$M range, otamixaban's IC50 is two or three orders of magnitude larger than the IC50 of camostat or nafamostat, respectively (cf. Table 1). However, in human PCLS, inhibitory effects of otamixaban and camostat were comparable (Fig. 1C).

In this context, Figure 1A shows the chemical structure of otamixaban and Figures 1B and 1C show the antiviral activity of otamixaban. Figure 1B displays the inhibition of entry driven by SARS-2-S (solid lines 1, 2, 3) or VSV-G (control, dashed lines) was analyzed using VSV pseudotyped particles. The average of three biological replicates $\pm$ standard deviation (SD) is shown. Figure 1C shows the Inhibition of SARS-CoV-2 infection of precision cut human lung slices (PCLS) by otamixaban (bars 1), camostat (bars 2) and nafamostat (bars 3), wherein PCLS were derived from a single donor. The mean results of three technical replicates are shown. Error bars indicate the SD.

Table 1: IC50 values and combination indices with uncertainty estimates for single and combination preparations of otamixaban, camostat, and nafamostat in Calu-3 cells. Combination indices (CI) were computed at 50% inhibition; the experiments were conducted at constant supplemental drug concentration (a: 1 nM [camostat], b: 0.1 nM [nafamostat])."

| Drug(s) | IC50 / nM | $\Delta$IC50 / nM | CI | $\Delta$CI |
|---|---|---|---|---|
| Otamixaban | 18.7 x 10³ | 562.1 | | |
| Camostat | 151.1 | 12.1 | | |
| Nafamostat | 11.8 | 1.2 | | |
| Otamixaban + camostat[a] | 758.2 | 192.4 | 0.040 | 0.010 |

(continued)

| Drug(s) | IC50 / nM | $\triangle$IC50 / nM | CI | $\triangle$CI |
|---|---|---|---|---|
| Otamixaban + nafamostat[b] | 728.5 | 115.7 | 0.039 | 0.006 |

In a subsequent step, the inventors tested otamixaban in the Calu-3 cell line in a combination preparation with constant small doses of camostat (1 nM) or nafamostat (0.1 nM), which are two orders of magnitude below their IC50 values (cf. Table 1). Surprisingly, it was found that the dose-response behavior of otamixaban in combination with either drug is significantly enhanced. E.g., at a drug concentration of 10 $\mu$M, SARS-CoV-2 cell entry is inhibited by 80% using otamixaban with camostat or nafamostat supplement compared to only 36% inhibition in case of otamixaban without supplement.

To quantify potential synergistic effects between the drugs, the combination index (CI) established by Chou and Talalay [31] was applied. The CI classifies drug interactions into additive (CI = 1), synergistic (CI < 1), and antagonistic (CI > 1). It was found that, at 50% inhibition, the CI is significantly smaller than 0.1 for both tested drug combinations (cf. Table 1), indicating that i) otamixaban and ii) camostat or nafamostat show very strong synergism [32].

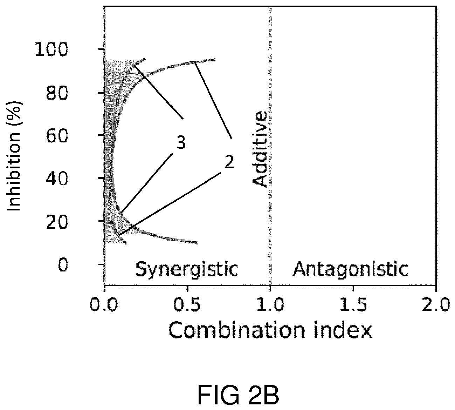

Figures 2A and 2B show the synergistic antiviral activity of otamixaban combined with camostat and nafamostat. Figure 2A shows the antiviral activity of otamixaban in combination with 1 nM camostat (curve 2) or 0.1 nM nafamostat (curve 3) compared with otamixaban alone (curve 1) analyzed using Calu-3 cells and VSV pseudotypes harboring either SARS-2-S (solid lines) or VSV-G (dashed lines) as described for Figure 1B. Corresponding IC50 values are highlighted with dotted lines and annotated on the left. The average of three biological replicates $\pm$ SD is shown.

Figure 2B shows the combination index as a function of inhibition strength for otamixaban plus 1 nM camostat (curve 2) and otamixaban plus 0.1 nM nafamostat (curve 2). The dashed line at CI = 1.0 indicates additivity (neither synergism nor antagonism). Combination indices below the dotted line indicate synergism (CI < 1). The inhibition range with strong synergism (IC < 0.3) is marked by shaded areas.

In fact, a simulation of the CI in the range between 10 and 95 % inhibition (Fig. 2B) shows that the majority of predicted CI values can be considered strongly synergistic (< 0.3), following the classification defined by Chou [32]. It should be noted that the CI simulation depends on the learnt parameters from the dose-response data, i.e. the prediction quality is expected to be high around 50 % inhibition. Peripheral deviations between camostat and nafamostat supplementation might therefore be an artifact of sparse sampling in that area.

Due to this unprecedented synergistic effect, the dose of otamixaban can be significantly reduced when used in combination with camostat or nafamostat. Specifically, the dose reduction index (DRI) of otamixaban at 50% inhibition is 24.7 and 25.7 for the two combination preparations, respectively. In other words, to achieve 50 % inhibition, only ~1/25 (~1/26) of the pure otamixaban concentrations are necessary when supplemented with nanomolar (subnanomolar) concentrations of camostat (nafamostat).

Analysis of molecular dynamic simulations (a set of 109 trajectories of 1 $\mu$s each) revealed that otamixaban binds TMPRSS2 with multiple metastable binding modes. In the most populated state, otamixaban is bound to the S1 pocket with the charged benzamidine moiety interacting with Asp435. This interaction is fundamental for the recognition of natural substrates and is typically exploited by several serine protease inhibitors, among which camostat and nafamostat can be found, both with a charged guanidinobenzoyl group instead of the benzamidine group of otamixaban [3]. Hence, the binding site is shared among the two drugs in the tested combination preparations, indicating that the synergistic effects described above are not likely to arise from simultaneous binding. At the opposite site of the benzamidine group, otamixaban has a long, hydrophobic pyridine-N-oxide moiety, with a polar head that is neutral at physiological pH (cf. Figure 1a). This moiety was found to mainly interact with the hydrophobic CH2 chains of Glu299 and Lys300, as well as the catalytic His296.

The second state is comparable to the crystal structure of otamixaban with its original target, the coagulation factor Xa (fXa) [25, 33]. It was found that the S1 interactions with the benzamidine moiety (in particular with Asp435) are equivalent, with a subtle difference in the amidinium ring conformation. Furthermore, the interactions of the opposite head differ as this moiety is designed to bind the fXa S4 pocket, a hydrophobic cavity formed by residues Tyr99, Phe174, and Trp215. In TMPRSS2, however, this cavity is less defined since the residue equivalent to Tyr99 is charged (Lys342), and the loop containing the residue equivalent to Phe174 is two amino acids longer and highly flexible (Tyr416-Asp417-Asn418).

These differences may explain why small modifications in the pyridine-N-oxide moiety can exert a large impact on drug's selectivity across different serine proteases [34]. The inventors further analyzed the contacts that otamixaban forms with TMPRSS2, finding that it mainly blocks the catalytic region, impeding the binding of any substrate that could be processed.

Finally, it is interesting to note that otamixaban has two potential hydrolyzable centers, an ester and an amide, that could be in principle digested by TMPRSS2. Nonetheless, otamixaban is known to be a reversible non-covalent inhibitor, with the two chemical groups being stable in blood and urine [34, 35]. This prompts the question why these two chemical groups are stable within the active site of the enzyme. To address this question, the inventors searched for conformations fulfilling certain structural criteria that are suitable for catalysis, including the integrity of the catalytic machinery (Ser441-His296 H-bond), the nucleophilic attack (Ser441-reactive center), and the position of part of the substrate inside the oxyanion hole (formed by Gly439 and Ser441 NH groups). For the amide group, the inventors found that it is never near the catalytic serine at the same time that it places its carboxylic group inside the oxyanion hole, two factors that are crucial to enhance the enzymatic activity [36]. Even if these two criteria were met, the inventors note that the N end of the amide is placed within the S1 pocket, inverted with respect to the orientation of natural substrates, which may further slow down a potential reaction. For the ester group, instead, reactive conformations were found, but just a very small fraction (0.13 %) of the total, suggesting that hydrolysis could be possible, but it will be minor. These factors may explain why these two chemical groups of otamixaban are preserved within the enzymatic cavity, rendering a stable, non-covalent reversible inhibitor.

## Discussion

The ongoing COVID-19 pandemic requires the development of potent drugs against SARS-CoV-2. A possible treatment strategy is to target the host cell protease TMPRSS2, an essential compound for viral host cell entry. In this application, the inventors identify otamixaban, a drug candidate admitted to phase III clinical trials, as a very promising TMPRSS2 inhibitor.

The inventors could show that even though otamixaban is only a weak TMPRSS2 inhibitor in cell culture, it is as potent as camostat in human lung tissue. One could argue that this intriguing result may arise from the fact that camostat is rapidly converted to a metabolite (GBPA) in the human body, which is slightly less potent [21]. However, the difference in potency between camostat and its metabolic product is likely not large enough to explain the leveling out of otamixaban and camostat observed in lung tissue. Thus, it seems more plausible that the presence of otamixaban's primary target fXa in PCLS but not in Calu-3 cells might be at the root of the observed differences.

Very surprisingly, otamixaban shows strong synergistic effects with camostat or nafamostat. This implies that otamixaban doses can be significantly lowered by adding (sub-) nanomolar concentrations of known TMPRSS2 inhibitors camostat or nafamostat.

Given that these three drugs bind to the S1 pocket of TMPRSS2 (as shown above and in Ref. [3]), it seems unlikely that they have a cooperative mechanism towards the same target. These observations suggest that the synergistic effect between otamixaban and camostat/nafamostat in Calu-3 cells is likely not due to drug-drug interactions or TMPRSS2 allosteric effects. Instead it could be explained by a secondary target. Additional investigation is necessary to shed light upon the precise mechanism of synergy between the drugs.

Otamixaban's potency in combination preparations is also encouraging from a therapeutic point of view: COVID-19 patients often suffer from hypercoagulation [37] and otamixaban was originally optimized for inhibiting coagulation factor Xa [25]. It might thus inhibit a suitable target orthogonal to TMPRSS2, possibly mitigating severe symptoms of COVID-19.

Having arrived at the phase III of clinical trials, the safety and pharmacokinetics of otamixaban have already been characterized. Across a 100-fold dose range, otamixaban has proven to be well tolerated in a cohort of healthy subjects [38]. It also showed rapid plasma distribution and elimination, with low variability of plasma exposure across subjects [35].

The availability of clinical data alongside the synergistic effects described in this application indicate that otamixaban in combination with nafamostat or camostat is a promising candidate for COVID-19 clinical trials.

## Materials and Methods

**High throughput TMPRSS2 activity assay.** A high throughput activity assay has been developed in order to test potential inhibitors of TMPRSS2 [29]. The experiment was performed in a 1536-well black plate according to the published protocol: Boc-Gln-Ala-Arg-AMC substrate (20 nL) and test compound (20 nL in DMSO) were added using an ECHO 655 acoustic dispenser (LabCyte). TMPRSS2 (150 nL) diluted in assay buffer (50 mM Tris pH 8, 150 mM NaCl, 0.01% Tween20) was dispensed to that, using a BioRAPTR (Beckman Coulter), for a total assay volume of 5 $\mu$L. After incubation at room temperature for 1 h, fluorescence was measured.

In order to detect inhibitors causing fluorescence quenching (those having a concentration-dependent decrease on AMC fluorescence) a counter-assay was performed as previously described [29]. 7-amino-4-methylcoumarin (20 nL) and inhibitor or DMSO (20 nL) were added using an ECHO 655 acoustic dispenser (LabCyte). Assay buffer (50 mM Tris pH 8, 150 mM NaCl, 0.01% Tween20) was added to that, for a total reaction volume of 5 $\mu$L.

PHERAstar with excitation at 340 nm and emission at 440 nm was used for detection and fluorescence was normalized relative to substrate alone (negative control) and substrate with 1 $\mu$M of nafamostat (positive control).

**Hit confirmation TMPRSS2 activity assay.** Compound dilutions ranging from 100 pM to 100 $\mu$M were prepared in assay buffer (50 mM Tris-HCI pH 8.0, 154 mM NaCl) containing a final DMSO concentration of 1%. In addition, recombinant TMPRSS2 (LifeSpan Biosciences, cat. no. LS-G57269) and the peptide substrate Boc-Gln-Ala-Arg-MCA (Peptide Institute, Inc., cat. no. 3135-v) were reconstituted in assay buffer, yielding concentrations of 2 $\mu$g/ml and 100 mM, respectively. Compound dilutions (25 $\mu$l/well, final concentration ranging from 50 pM to 50 $\mu$M) and recombinant TMPRSS2 (25 $\mu$l/well, final concentration 1 $\mu$g/ml) were mixed in black 96-well plates and incubated for 10 min. Next, assay buffer containing 20 $\mu$M Boc-Gln-Ala-Arg-MCA peptide substrate (50 $\mu$l/well, final concentration 10 $\mu$M) was added and samples were incubated for 3 h at 37 °C before fluorescence intensity was recorded using the Tecan Genios plate reader and the Magellan V6.4 Software (Tecan; excitation: 360 nm, emission: 465 nm).

In order to define the upper and lower limits of the assay, the following controls were used: (i) to determine maximum TMPRSS2 enzyme activity, recombinant TMPRSS2 (25 $\mu$l/well, final concentration 1 $\mu$g/ml) was pre-incubated with assay buffer containing 1% DMSO but no compound (25 $\mu$l/well) before the peptide substrate (50 $\mu$l/well, final concentration 10 $\mu$M) was added; (ii) to determine the unspecific background of the assay, peptide substrate (50 $\mu$l/well, final concentration 10 $\mu$M) was incubated with assay buffer containing 0.5% DMSO (50 $\mu$l/well) in the absence of both recombinant TMPRSS2 and compound.

**Cell culture.** All cell lines were incubated at 37 °C in a humidified atmosphere with 5% $CO_2$. Vero E6 (African green Monkey kidney; ATCC no. CRL-1586; kindly provided by Marcel A. Müller and Christan Drosten, Institute of Virology, Charité - Universitätsmedizin Berlin, Germany) and HEK-293T (human embryonic kidney; DSMZ no. ACC 635) cells were cultivated in Dulbecco's modified Eagle medium containing 10% fetal bovine serum (FCS, Biochrom), 100 U/ml of penicillin and 0.1 mg/ml of streptomycin (PAN-Biotech), while Calu-3 cells (human lung adenocarcinoma; kindly provided by Stephan Ludwig, Institute of Virology, University of Münster, Germany) were cultivated in minimum essential medium supplemented with 10% FCS, 100 U/ml of penicillin and 0.1 mg/ml of streptomycin (PAN-Biotech), 1x non-essential amino acid solution (from 100x stock, PAA) and 1 mM sodium pyruvate (Thermo Fisher Scientific). Transfection of HEK-293T was carried out by calcium-phosphate precipitation.

**Plasmids.** The expression plasmids for severe acute respiratory syndrome coronavirus 2 spike glycoprotein (SARS-2-S) and vesicular stomatitis virus glycoprotein (VSV-G) that were used to generate VSV pseudotype parti-cles harboring the respective viral proteins have been described elsewhere [4, 39]. Further, empty pCG1 expression plasmid (kindly provided by Roberto Cattaneo, Mayo Clinic College of Medicine, Rochester, MN, USA) was used for the production of VSV particles bearing no viral protein.

**Reconstitution of compounds and storage.** Camostat mesylate (Tocris, cat. no. 3193), nafamostat mesylate (Tocris, cat. no. 3081) and otamixaban (AdooQ BioScience, cat. no. A11517) were reconstituted in dimethyl sulfoxide (DMSO), yielding stock concentrations of 10 mM. For each compound stock multiple small volume aliquots were prepared and stored at -20 °C. Of note, in order to avoid changes in compound activity due to multiple freeze-thaw cycles, residual compound from thawed stocks was discarded. Please note that compounds for the high throughput activity assay were treated in a separate facility (cf. High throughput TMPRSS2 activity assay).

**Analysis of SARS-2-S-driven cell entry using VSV pseudotypes.** VSV pseudotype particles bearing SARS-2-S, VSV-G or no viral protein (negative control) were produced according to an established protocol [40]. In brief, HEK-293T cells transfected to express the respective viral protein or no viral protein were inoculated with a replication-deficient VSV vector that lacks the genetic information for VSV-G and instead possesses individual open reading frames for an enhanced green fluorescent protein and firefly luciferase (FLuc), VSVΔG-FLuc (kindly provided by Gert Zimmer, Institute of Virology and Immunology, Mittelhäusern, Switzerland) [41]. Following an incubation period of 1 h at 37 °C, the cells were washed and received fresh culture medium. In case of cells transfected with SARS-2-S expression vector or empty plasmid, the medium was further supplemented with anti-VSV-G antibody (culture supernatant from I1-hybridoma cells; ATCC no. CRL-2700). At 16-18 h post inoculation, the culture supernatant containing the VSV pseudotype particles was collected, clarified from cellular debris by centrifugation (2,000 x g, 10 min, room temperature) and stored at -80 °C until further use. In order to analyze the effects of the tested compounds on SARS-2-S-driven VSV pseudotype entry into target cells, Calu-3 cells, which were grown in 96-well plates, were incubated for 1 h at 37 °C in the presence of different concentrations of the respective compounds or solvent (DMSO) diluted in culture medium (50 $\mu$l/well). Next, VSV pseu-dotypes were added (50 $\mu$l/well) and cells were further incubated for 16-18 h, before the efficiency of pseudotype entry was assessed. For this, the culture supernatant was aspirated and cells were lysed by incubation with 1x Cell Culture Lysis Reagent (produced from 5-fold concentrated stock, Promega) for 30 min at room temperature. Thereafter, lysates were transferred into white 96-well plates. Finally, FLuc activity was recorded using a Hidex Sense plate luminometer (Hidex) following addition of a commercial substrate (Beetle-Juice, PJK).

**Infection of ex vivo human lung cultures with authentic SARS-CoV-2 and quantification of viral titer.** Experiments with human lung tissue were approved by the Ethics Committee of the Hannover Medical School (MHH, Hannover, Germany) and are in compliance with The Code of Ethics of the World Medical Association (number 2701-2015). All patients or their next of kin gave written informed consent for the use of lung tissue for research. Ex vivo human lung cultures (precision cut lung slices, PCLS) were prepared according to a published protocol [42]. Only macroscopically disease-free parts of lung tissue obtained from lung cancer patients that underwent lobe resection at the Hannover Medical School (MHH, Hannover, Germany) were used for experiments. First, the lung lobe was inflated with DMEM/F-12 medium containing 2% agarose. Following agarose polymerization, the tissue was cut into slabs and tissue cores of 8 mm in diameter were cut into 300 $\mu$m thin slices. Cultivation of PCLS was carried out at 37 °C and 5% $CO_2$ using Ham's F-12 medium containing 0.1 mg/ml streptomycin, 10 U/ml penicillin, 0.05 mg/ml gentamicin and 0.01 mg/ml enrofloxacin. For infection experiments, only PCLS with a ciliary activity of 75% or higher were used. The PCLS were placed into 48-well plates and incubated for 1 h at 37 °C and 5% $CO_2$ in the presence of different concentrations of the respective compounds (PCLS treated with DMSO served as control). Next, the culture medium was removed and PCLS were inoculated with SARS-CoV-2 isolate hCoV-19/Germany/FI1103201/2020 (kindly provided by Stephan Ludwig, Institute of Virology, University of Münster, Germany) using an infectious dose of 4 x 10$^5$ pfu/ml (250 $\mu$l/well). At 1 h post inoculation, the inoculum was removed and PCLS were washed three times with phosphate-buffered saline (PBS), before culture medium (500 $\mu$l/well) containing the respective inhibitor or DMSO was added. Samples (100 $\mu$l) were collected at 1 h (washing control) and 24 h (end point) post infection and stored at -80 °C for virus titration. Viral titers were determined by plaque titration. For this, Vero E6 cells that were grown in 12-well plates were inoculated with serial dilutions of supernatant (inoculum = 850 $\mu$l) and incubated for 1 h at 37 °C and 5% $CO_2$. Thereafter, the inoculum was removed and cells were washed with PBS. Next, cells were overlaid with Eagle's minimal essential medium without phenol red (Lonza) containing 1% plaque agarose (Biozym) and incubated for 72 h at 37 °C and 5% $CO_2$, before plaques were counted to determine viral titers (given as plaque forming units per ml, pfu/ml).

**Docking.** Due to the lack of a crystal structure for TMPRSS2, the inventors manually extracted 10 representative frames from a precursive molecular dynamics (MD) dataset [3] which was originally seeded with a homology model [27]. The MD construct includes only the protease domain (amino acids 256 to 491). Even though TMPRSS2 has a transmembrane domain, the catalytic domain (i.e, the inventor's MD setup) itself is not embedded or directly linked to the membrane, nor is there evidence of lipids playing a role in catalysis. The inventors therefore assume that a simulation in solvent is a realistic model.

For the ligand structure, the inventors downloaded the three-dimensional representation of the reference molecule from the ZINC database (ZINC ID: ZINC000001908051) [43]. The inventors prepared both the receptor and the ligand structures with MGLTools [44].

The inventors performed protein-ligand docking using smina [45], a fork of AutoDock Vina [46]. The search space was defined as a box of 30 Å$^3$ centered on the C$\alpha$ atom of the catalytic serine (Ser441). The inventors identified five important residues whose rigidity could negatively influence ligand binding into the S1 pocket, namely: Glu299, Lys300, Asp435, Gln438 and Trp461. The side-chains of these residues were kept flexible throughout the run in order to explore their different conformations. The inventors used the Vinardo scoring function [47] and the default exhaustiveness of 10.

For each receptor structure docked against the ligand, the inventors took the single highest-scored binding pose. Finally, the inventors used the SMILES to determine the positions of missing hydrogens (omitted by smina) and added them back to the docking poses.

**Molecular dynamics simulations.** MD simulations were run with OpenMM 7.4.0 [48] with the amber14SB forcefield [49] for protein and openff-1.1.0 [50] for the ligand parameters. The inventors initiated a simulation box of side length 7.2 nm, used the TIP3P water model [51] with a NaCl ion concentration of 0.1 mol/l at neutral charge. The setups contained between 10354 and 10429 water molecules, depending on the docking pose and TMPRSS2 conformation.

The inventors ran simulations in the NPT ensemble. The temperature was kept at 310 K (physiological temperature) and the pressure at 1 bar. Rigid water molecules, PME electrostatics, and a 1 nm cutoff for non-bonded interactions were used. The inventors further applied a Langevin integrator with 4 fs integration step using hydrogen mass repartitioning (HMR, 4 amu hydrogen mass). HMR slows down covalent hydrogen bond vibrations such that a larger integration step is possible while conserving the thermodynamics and kinetics [52, 53]. The trajectories accumulate to 109 $\mu$s, each containing a simulation time of 1 $\mu$s. VMD [54] was used to visualize structures.

MD simulations were seeded from the docked structures, as described above. More specifically, the inventors selected as starting structures the four docking poses where the ligand had identical chirality with respect to the molecule in the ZINC database (ZINC ID: ZINC000001908051). Please note, again, that the protein structures are based on a homology model [27] as detailed in Ref. [3].

The inventors analyzed MD simulations by using inverse minimal distances between the different drug groups (benzamidine-group, methyl-ester, pyridine-N-oxid, methyl-group) to each protein residue with PyEMMA 2.5.7 [55]. The inventors further performed a linear VAMP [56] dimension reduction operation with a lag time of 5 ns, used 10 dimensions with the highest kinetic variance, and performed a regular spatial clustering with a minimal distance of 0.9. The discrete state with the most population was subsequently estimated by the argmax of a histogram.

Based on the same VAMP projection, the inventors used a different discretization (k-means algorithm with 300 cluster centers) to estimate a 16 state hidden Markov model [57] (HMM) at lag time 2 ns. The HMM model is used to assign metastable states (binding modes) and to extract metastable trajectories (Viterbi algorithm [58]). The inventors used the metastable trajectories to verify that the model captures the metastable dynamics and for assessing empirical state probabilities through histogram counting.

**Dose-response curve fitting.** The inventors normalized the inhibition efficiency against the respective control(s) for each of the experiments. The inventors then used the generalized four-parameter log-logistic model to fit the dose-response curves on the normalized data. The model is defined as:

$$f\big(x, (b, c, d, e)\big) = c + \frac{d-c}{1+\exp(b \cdot (\ln(x)-\ln(e)))} \qquad (1)$$

where x is the concentration of the inhibitor, b is the Hill slope, c and d are the lower and the upper limit, respectively, and e is the IC50 value. The upper and the lower limit were set to 0 and 100, respectively. The inventors used the curve-fitting algorithm present in the scipy package [59] to obtain the Hill slope and the IC50 value, with their associated error estimates.

**Drug combination analysis.** The combination index (CI) for two mutually exclusive drugs was computed using the following equation [31]

$$CI = \frac{[D]_1}{[D_x]_1} + \frac{[D]_2}{[D_x]_2}, \qquad (2)$$

where $[D_x]_i$ and $[D]_i$ are the concentrations of drug i when acting alone and in combination preparation, respectively. It is evaluated for a given percentage of inhibition (of single and combination preparations) and quantifies how potent the drug combinations are with respect to the sum of their constituents. The inventors used Gaussian error propagation to estimate the uncertainty of this quantity.

Since it is *a priori* unclear whether the drugs in the combination preparations are mutually exclusive or not, the inventors have computed the correction terms for mutually non-exclusive drugs, $\frac{[D]_1[D]_2}{[D_x]_1[D_x]_2}$, and found them to be negligibly small for both combination preparations.

To estimate the CI for different inhibition values, the inventors have analytically inverted the dose-response function (Eq. 1). It is evaluated from 10 to 95% inhibition, i.e. in the range in which the experimental measurements allow a reasonable curve fit.

The dose reduction index (DRI) for drug *i* is calculated as [32]:

$$DRI_i = \frac{[D_x]_i}{[D]_i} \cdot (3)$$

**List of references cited in the preceding sections**

[1] B. Hu, H. Guo, P. Zhou, and Z.-L. Shi. "Characteristics of SARS-CoV-2 and COVID-19". Nat. Rev. Microbiol. (2020).

[2] The World Health Organization. Weekly Epidemiological Update, Coronavirus disease 2019 (COVID-19). 9 March 2021.

[3] T. Hempel et al. "Molecular mechanism of inhibiting the SARS-CoV-2 cell entry facilitator TMPRSS2 with camostat and nafamostat". Chemical Science (2021).

[4] M. Hoffmann et al. "SARS-CoV-2 Cell Entry Depends on ACE2 and TMPRSS2 and Is Blocked by a Clinically Proven Protease Inhibitor". Cell 181.2 (2020), 271-280.e8.

[5] B. A. Schuler et al. Age-Determined Expression of Priming Protease TMPRSS2 and Localization of SARS-CoV-2 Infection in the Lung Epithelium. Preprint. bioRxiv doi:10.1101/2020.05.22.111187.2020.

[6] M. Montopoli et al. "Androgen-Deprivation Therapies for Prostate Cancer and Risk of Infection by SARS-CoV-2: A Population-Based Study (N = 4532)". Ann. Oncol. (2020).

[7] M. Hoffmann et al. "Chloroquine does not inhibit infection of human lung cells with SARS-CoV-2". Nature (2020).

[8] T. Ou et al. Hydroxychloroquine-mediated inhibition of SARS-CoV-2 entry is attenuated by TMPRSS2. bioRxiv doi:10.1101-2020.07.22.216150. 2020.

[9] E. R. Kastenhuber et al. Coagulation factors directly cleave SARS-CoV-2 spike and enhance viral entry. bioRxiv doi:10.1101/2021.03.31.437960. 2021.

[10] S. Matsuyama et al. "Efficient Activation of the Severe Acute Respiratory Syndrome Coronavirus Spike Protein by the Transmembrane Protease TMPRSS2". J. Virol. 84.24 (2010), pp. 12658-12664.

[11] A. Shulla et al. "A Transmembrane Serine Protease Is Linked to the Severe Acute Respiratory Syndrome Coronavirus Receptor and Activates Virus Entry". J. Virol. 85.2 (2010), pp. 873-882.

[12] I. Glowacka et al. "Evidence that TMPRSS2 Activates the Severe Acute Respiratory Syndrome Coronavirus Spike Protein for Membrane Fusion and Reduces Viral Control by the Humoral Immune Response". J. Virol. 85.9 (2011), pp. 4122-4134.

[13] S. Bertram et al. "Cleavage and Activation of the Severe Acute Respiratory Syndrome Coronavirus Spike Protein by Human Airway Trypsin-Like Protease". J. Virol. 85.24 (2011), pp. 13363-13372.

[14] S. Gierer et al. "The Spike Protein of the Emerging Betacoronavirus EMC Uses a Novel Coronavirus Receptor for Entry, Can Be Activated by TMPRSS2, and Is Targeted by Neutralizing Antibodies". J. Virol. 87.10 (2013), pp. 5502-5511.

[15] S. Bertram et al. "TMPRSS2 Activates the Human Coronavirus 229E for Cathepsin-Independent Host Cell Entry and Is Expressed in Viral Target Cells in the Respiratory Epithelium". J. Virol. 87.11 (2013), pp. 6150-6160.

[16] K. Shirato, M. Kawase, and S. Matsuyama. "Middle East Respiratory Syndrome Coronavirus Infection Mediated by the Transmembrane Serine Protease TMPRSS2". J. Virol. 87.23 (2013), pp. 12552-12561.

[17] E. Böttcher et al. "Proteolytic Activation of Influenza Viruses by Serine Proteases TMPRSS2 and HAT from Human Airway Epithelium". J. Virol. 80.19 (2006), pp. 9896-9898.

[18] C. Chaipan et al. "Proteolytic Activation of the 1918 Influenza Virus Hemagglutinin". J. Virol. 83.7 (2009), pp. 3200-3211.

[19] K. Sakai et al. "The Host Protease TMPRSS2 Plays a Major Role in in Vivo Replication of Emerging H7N9 and Seasonal Influenza Viruses". J. Virol. 88.10 (2014), pp. 5608-5616.

[20] T. S. Kim, C. Heinlein, R. C. Hackman, and P. S. Nelson. "Phenotypic Analysis of Mice Lacking the Tmprss2-Encoded Protease". Mol. Cell. Biol. 26.3 (2006), pp. 965-975.

[21] M. Hoffmann et al. "Camostat Mesylate Inhibits SARS-CoV-2 Activation by TMPRSS2-Related Proteases and Its Metabolite GBPA Exerts Antiviral Activity". EBioMedicine (2021), p. 103255.

[22] M. Hoffmann et al. "Nafamostat Mesylate Blocks Activation of SARS-CoV-2: New Treatment Option for COVID-19". Antimicrob. Agents Chemother. 64.6 (2020).

[23] N. P. Azouz et al. Alpha 1 Antitrypsin is an Inhibitor of the SARS-CoV-2-Priming Protease TMPRSS2. bioRxiv doi:10.1101/2020.05.04.077826. 2020.

[24] D. Bestle et al. "TMPRSS2 and furin are both essential for proteolytic activation of SARS-CoV-2 in human airway cells". Life Science Alliance 3.9 (2020), e202000786.

[25] K. R. Guertin et al. "Optimization of the β-Aminoester class of factor Xa inhibitors. part 2: Identification of FXV673 as a potent and selective inhibitor with excellent In vivo anticoagulant activity". Bioorganic Med. Chem. Lett. 12.12 (2002), pp. 1671-1674.

[26] P. G. Steg. "Anticoagulation With Otamixaban and Ischemic Events in Non-ST-Segment Elevation Acute Coronary Syndromes". JAMA 310.11 (2013), p. 1145.

[27] S. Rensi et al. Homology Modeling of TMPRSS2 Yields Candidate Drugs That May Inhibit Entry of SARS-CoV-2 into Human Cells. Preprint. chemRxiv doi:10.26434/chemrxiv.12009582. 2020.

[28] X. Hu et al. Non-covalent TMPRSS2 inhibitors identified from virtual screening. bioRxiv doi:10.1101/2020.12.28.424413. 2020.

[29] J. H. Shrimp et al. "An Enzymatic TMPRSS2 Assay for Assessment of Clinical Candidates and Discovery of Inhibitors as Potential Treatment of COVID-19". ACS Pharmacology & Translational Science 3.5 (2020), pp. 997-1007.

[30] W. Sun, P. E. Sanderson, and W. Zheng. "Drug combination therapy increases successful drug repositioning". Drug Discovery Today 21.7 (2016), pp. 1189-1195.

[31] T.-C. Chou and P. Talalay. "Quantitative analysis of dose-effect relationships: the combined effects of multiple drugs or enzyme inhibitors". Advances in Enzyme Regulation 22 (1984), pp. 27-55.

[32] T.-C. Chou. "Theoretical Basis, Experimental Design, and Computerized Simulation of Synergism and Antagonism in Drug Combination Studies". Pharmacological Reviews 58.3 (2006), pp. 621-681.

[33] V. Chu et al. "Pharmacological Characterization of a Novel Factor Xa Inhibitor, FXV673". Thrombosis Research 103.4 (2001), pp. 309-324.

[34] K. Guertin and Y.-M. Choi. "The Discovery of the Factor Xa Inhibitor Otamixaban: From Lead Identification to Clinical Development". Current Medicinal Chemistry 14.23 (2007), pp. 2471-2481.

[35] A. Paccaly et al. "Pharmacokinetics of Otamixaban, a Direct Factor Xa Inhibitor, in Healthy Male Subjects: Pharmacokinetic Model Development for Phase 2/3 Simulation of Exposure". The Journal of Clinical Pharmacology 46.1 (2006), pp. 37-44.

[36] L. Hedstrom. "Serine Protease Mechanism and Specificity". Chemical Reviews 102.12 (2002), pp. 4501-4524.

[37] M. Y. Abou-Ismail, A. Diamond, S. Kapoor, Y. Arafah, and L. Nayak. "The hypercoagulable state in COVID-19: Incidence, pathophysiology, and management". Thrombosis Research 194 (2020), pp.101-115.

[38] A. Paccaly et al. "Pharmacodynamic markers in the early clinical assessment of otamixaban, a direct factor Xa inhibitor". Thrombosis and Haemostasis 94.12 (2005), pp. 1156-1163.

[39] C. Brinkmann et al. "The Glycoprotein of Vesicular Stomatitis Virus Promotes Release of Virus-like Particles from Tetherin-Positive Cells". PLoS One 12.12 (2017), e0189073.

[40] H. Kleine-Weber et al. "Mutations in the Spike Protein of Middle East Respiratory Syndrome Coronavirus Transmitted in Korea Increase Resistance to Antibody-Mediated Neutralization". J Virol 93.2 (2019).

[41] M. Berger Rentsch and G. Zimmer. "A Vesicular Stomatitis Virus Replicon-Based Bioassay for the Rapid and Sensitive Determination of Multi-Species Type I Interferon". PLoS One 6.10 (2011), e25858.

[42] V. Neuhaus et al. "Assessment of the Cytotoxic and Immunomodulatory Effects of Substances in Human Precision-Cut Lung Slices". J Vis Exp 135 (2018).

[43] T. Sterling and J. J. Irwin. "ZINC 15 - Ligand Discovery for Everyone". Journal of Chemical Information and Modeling 55.11 (2015), pp. 2324-2337.

[44] G. M. Morris et al. "AutoDock4 and AutoDockTools4: Automated docking with selective receptor flexibility". Journal of Computational Chemistry 30.16 (2009), pp. 2785-2791.

[45] D. R. Koes, M. P. Baumgartner, and C. J. Camacho. "Lessons Learned in Empirical Scoring with smina from the CSAR 2011 Benchmarking Exercise". Journal of Chemical Information and Modeling 53.8 (2013), pp. 1893-1904.

[46] O. Trott and A. J. Olson. "AutoDock Vina: Improving the speed and accuracy of docking with a new scoring function, efficient optimization, and multithreading". Journal of Computational Chemistry (2009).

[47] R. Quiroga and M. A. Villarreal. "Vinardo: A Scoring Function Based on Autodock Vina Improves Scoring, Docking, and Virtual Screening". PLOS ONE 11.5 (2016), e0155183.

[48] P. Eastman et al. "OpenMM 7: Rapid Development of High Performance Algorithms for Molecular Dynamics". PLOS Comput. Biol. 13.7 (2017), e1005659.

[49] J. A. Maier et al. "ff14SB: Improving the Accuracy of Protein Side Chain and Backbone Parameters from ff99SB". J. Chem. Theory Comput. 11.8 (2015), pp. 3696-3713.

[50] Y. Qiu et al. "Development and Benchmarking of Open Force Field v1.0.0, the Parsley Small Molecule Force Field" (2020).

[51] W. L. Jorgensen, J. Chandrasekhar, J. D. Madura, R. W. Impey, and M. L. Klein. "Comparison of Simple Potential Functions for Simulating Liquid Water". J. Chem. Phys. 79.2 (1983), pp. 926-935.

[52] C. W. Hopkins, S. Le Grand, R. C. Walker, and A. E. Roitberg. "Long-Time-Step Molecular Dynamics through Hydrogen Mass Repartitioning". J. Chem. Theory Comput. 11.4 (2015), pp. 1864-1874.

[53] F. Rao and M. Spichty. "Thermodynamics and Kinetics of Large-Time-Step Molecular Dynamics". J. Comput. Chem. 33.5 (2012), pp. 475-483.

[54] W. Humphrey, A. Dalke, and K. Schulten. "VMD: Visual Molecular Dynamics". J. Mol. Graph. 14.1 (1996), pp. 33-38.

[55] M. K. Scherer et al. "PyEMMA 2: A Software Package for Estimation, Validation, and Analysis of Markov Models". J. Chem. Theory Comput. 11.11 (2015), pp. 5525-5542.

[56] H. Wu and F. Noé. "Variational Approach for Learning Markov Processes from Time Series Data". J Nonlinear Sci (2019).

[57] F. Noé, H. Wu, J.-H. Prinz, and N. Plattner. "Projected and Hidden Markov Models for Calculating Kinetics and Metastable States of Complex Molecules". J. Chem. Phys. 139.18 (2013), p. 184114.

[58] L. R. Rabiner. "A Tutorial on Hidden Markov Models and Selected Applications in Speech Recognition". Proc. IEEE 77.2 (1989), pp. 257-286.

[59] P. Virtanen et al. "SciPy 1.0: Fundamental Algorithms for Scientific Computing in Python". Nat. Methods 17 (2020), pp. 261-272.

**Claims**

1. Pharmaceutical composition comprising a pharmaceutically effective amount of i) otamixaban or a pharmaceutically acceptable salt thereof and ii) at least one of camostat, a pharmaceutically acceptable salt of camostat, nafamostat, and a pharmaceutically acceptable salt of nafamostat.

2. Pharmaceutical composition according to claim 1, **characterized in that** a ratio between i) the pharmaceutically effective amount of camostat, a pharmaceutically acceptable salt of camostat, nafamostat, or a pharmaceutically acceptable salt of camostat and ii) the pharmaceutically effective amount of otamixaban or a pharmaceutically acceptable salt thereof lies in a range of from 1:10000 to 1:1000.

3. Pharmaceutical composition according to claim 1 or 2, **characterized in that** the pharmaceutical composition comprises a pharmaceutically effective amount of i) otamixaban or a pharmaceutically acceptable salt thereof and ii) camostat or a pharmaceutically acceptable salt of camostat.

4. Pharmaceutical composition according to any of the preceding claims, **characterized in that** the pharmaceutical composition comprises a pharmaceutically effective amount of otamixaban and camostat.

5. Pharmaceutical composition according to any of the preceding claims, **characterized in that** the pharmaceutical composition comprises a pharmaceutically effective amount of i) otamixaban or a pharmaceutically acceptable salt thereof and ii) nafamostat or a pharmaceutically acceptable salt of nafamostat.

6. Pharmaceutical composition according to any of the preceding claims, **characterized in that** the pharmaceutical composition comprises a pharmaceutically effective amount of otamixaban and nafamostat.

7. Pharmaceutical composition according to any of the preceding claims for use in treating or preventing an infection with severe acute respiratory syndrome coronavirus 2 (SARS-CoV-2).

8. Pharmaceutical composition according to any of claims 1 to 6 for use in treating or preventing COVID-19.

9. Combination of i) otamixaban or a pharmaceutically acceptable salt thereof and ii) at least one of camostat, a pharmaceutically acceptable salt of camostat, nafamostat, and a pharmaceutically acceptable salt of nafamostat for use in treating or preventing an infection with severe acute respiratory syndrome coronavirus 2 (SARS-CoV-2).

10. Combination of i) otamixaban or a pharmaceutically acceptable salt thereof and ii) at least one of camostat, a pharmaceutically acceptable salt of camostat, nafamostat, and a pharmaceutically acceptable salt of nafamostat for use in treating or preventing COVID-19.

FIG 1A

FIG 1B

FIG 1C

FIG 2A

FIG 2B

Europäisches
Patentamt

European
Patent Office

Office européen
des brevets

# EUROPEAN SEARCH REPORT

Application Number

EP 21 18 7449

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HU XIN ET AL: "Discovery of TMPRSS2 Inhibitors from Virtual Screening as a Potential Treatment of COVID-19", ACS PHARMACOLOGY & TRANSLATIONAL SCIENCE, vol. 4, no. 3, 2 April 2021 (2021-04-02), pages 1124-1135, XP055870246, ISSN: 2575-9108, DOI: 10.1021/acsptsci.0c00221 Retrieved from the Internet: URL:https://pubs.acs.org/doi/pdf/10.1021/a csptsci.0c00221> * the whole document * | 1-10 | INV. A61K31/245 A61K31/4425 A61P31/14 |
| A | WANG XINLING ET AL: "Broad-Spectrum Coronavirus Fusion Inhibitors to Combat COVID-19 and Other Emerging Coronavirus Diseases", INTERNATIONAL JOURNAL OF MOLECULAR SCIENCES, vol. 21, no. 11, 1 January 2020 (2020-01-01), page 3843, XP055783052, Basel, CH ISSN: 1661-6596, DOI: 10.3390/ijms21113843 * page 7, paragraph 2 * | 1-10 | TECHNICAL FIELDS SEARCHED (IPC) A61K A61P |

The present search report has been drawn up for all claims

| Place of search | Date of completion of the search | Examiner |
|---|---|---|
| Munich | 9 December 2021 | Ganschow, Silke |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.........................................................................
& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **B. HU ; H. GUO ; P. ZHOU ; Z.-L. SHI.** Characteristics of SARS-CoV-2 and COVID-19. *Nat. Rev. Microbiol.,* 2020
- Weekly Epidemiological Update, Coronavirus disease 2019 (COVID-19). The World Health Organization, 09 March 2021
- **T. HEMPEL et al.** Molecular mechanism of inhibiting the SARS-CoV-2 cell entry facilitator TMPRSS2 with camostat and nafamostat. *Chemical Science,* 2021
- **M. HOFFMANN et al.** SARS-CoV-2 Cell Entry Depends on ACE2 and TMPRSS2 and Is Blocked by a Clinically Proven Protease Inhibitor. *Cell,* 2020, vol. 181.2, 271-280.e8
- **B. A. SCHULER et al.** Age-Determined Expression of Priming Protease TMPRSS2 and Localization of SARS-CoV-2 Infection in the Lung Epithelium. *Preprint. bioRxiv*
- **M. MONTOPOLI et al.** Androgen-Deprivation Therapies for Prostate Cancer and Risk of Infection by SARS-CoV-2: A Population-Based Study (N = 4532). *Ann. Oncol.,* 2020
- **M. HOFFMANN et al.** Chloroquine does not inhibit infection of human lung cells with SARS-CoV-2. *Nature,* 2020
- **T. OU et al.** Hydroxychloroquine-mediated inhibition of SARS-CoV-2 entry is attenuated by TMPRSS2. *bioRxiv,* 2020
- **E. R. KASTENHUBER et al.** Coagulation factors directly cleave SARS-CoV-2 spike and enhance viral entry. *bioRxiv,* 2021
- **S. MATSUYAMA et al.** Efficient Activation of the Severe Acute Respiratory Syndrome Coronavirus Spike Protein by the Transmembrane Protease TMPRSS2. *J. Virol.,* 2010, vol. 84.24, 12658-12664
- **A. SHULLA et al.** A Transmembrane Serine Protease Is Linked to the Severe Acute Respiratory Syndrome Coronavirus Receptor and Activates Virus Entry. *J. Virol.,* 2010, vol. 85.2, 873-882
- **I. GLOWACKA et al.** Evidence that TMPRSS2 Activates the Severe Acute Respiratory Syndrome Coronavirus Spike Protein for Membrane Fusion and Reduces Viral Control by the Humoral Immune Response. *J. Virol.,* 2011, vol. 85.9, 4122-4134
- **S. BERTRAM et al.** Cleavage and Activation of the Severe Acute Respiratory Syndrome Coronavirus Spike Protein by Human Airway Trypsin-Like Protease. *J. Virol.,* 2011, vol. 85.24, 13363-13372

- **S. GIERER et al.** The Spike Protein of the Emerging Betacoronavirus EMC Uses a Novel Coronavirus Receptor for Entry, Can Be Activated by TMPRSS2, and Is Targeted by Neutralizing Antibodies. *J. Virol.,* 2013, vol. 87.10, 5502-5511
- **S. BERTRAM et al.** TMPRSS2 Activates the Human Coronavirus 229E for Cathepsin-Independent Host Cell Entry and Is Expressed in Viral Target Cells in the Respiratory Epithelium. *J. Virol.,* 2013, vol. 87.11, 6150-6160
- **K. SHIRATO ; M. KAWASE ; S. MATSUYAMA.** Middle East Respiratory Syndrome Coronavirus Infection Mediated by the Transmembrane Serine Protease TMPRSS2. *J. Virol.,* 2013, vol. 87.23, 12552-12561
- **E. BÖTTCHER et al.** Proteolytic Activation of Influenza Viruses by Serine Proteases TMPRSS2 and HAT from Human Airway Epithelium. *J. Virol.,* 2006, vol. 80.19, 9896-9898
- **C. CHAIPAN et al.** Proteolytic Activation of the 1918 Influenza Virus Hemagglutinin. *J. Virol.,* 2009, vol. 83.7, 3200-3211
- **K. SAKAI et al.** The Host Protease TMPRSS2 Plays a Major Role in in Vivo Replication of Emerging H7N9 and Seasonal Influenza Viruses. *J. Virol.,* 2014, vol. 88.10, 5608-5616
- **T. S. KIM ; C. HEINLEIN ; R. C. HACKMAN ; P. S. NELSON.** Phenotypic Analysis of Mice Lacking the Tmprss2-Encoded Protease. *Mol. Cell. Biol.,* 2006, vol. 26.3, 965-975
- **M. HOFFMANN et al.** Camostat Mesylate Inhibits SARS-CoV-2 Activation by TMPRSS2-Related Proteases and Its Metabolite GBPA Exerts Antiviral Activity. *EBioMedicine,* 2021, 103255
- **M. HOFFMANN et al.** Nafamostat Mesylate Blocks Activation of SARS-CoV-2: New Treatment Option for COVID-19. *Antimicrob. Agents Chemother.,* 2020, vol. 64.6
- **N. P. AZOUZ et al.** Alpha 1 Antitrypsin is an Inhibitor of the SARS-CoV-2-Priming Protease TMPRSS2. *bioRxiv,* 2020
- **D. BESTLE et al.** TMPRSS2 and furin are both essential for proteolytic activation of SARS-CoV-2 in human airway cells. *Life Science Alliance,* 2020, vol. 3.9

- **K. R. GUERTIN et al.** Optimization of the β-Aminoester class of factor Xa inhibitors. part 2: Identification of FXV673 as a potent and selective inhibitor with excellent In vivo anticoagulant activity. *Bioorganic Med. Chem. Lett.,* 2002, vol. 12.12, 1671-1674
- **P. G. STEG.** Anticoagulation With Otamixaban and Ischemic Events in Non-ST-Segment Elevation Acute Coronary Syndromes. *JAMA,* 2013, vol. 310.11, 1145
- **S. RENSI et al.** Homology Modeling of TMPRSS2 Yields Candidate Drugs That May Inhibit Entry of SARS-CoV-2 into Human Cells. *Preprint. chemRxiv,* 2020
- **X. HU et al.** Non-covalent TMPRSS2 inhibitors identified from virtual screening. *bioRxiv,* 2020
- **J. H. SHRIMP et al.** An Enzymatic TMPRSS2 Assay for Assessment of Clinical Candidates and Discovery of Inhibitors as Potential Treatment of COVID-19. *ACS Pharmacology & Translational Science,* 2020, vol. 3.5, 997-1007
- **W. SUN ; P. E. SANDERSON ; W. ZHENG.** Drug combination therapy increases successful drug repositioning. *Drug Discovery Today,* 2016, vol. 21.7, 1189-1195
- **T.-C. CHOU ; P. TALALAY.** Quantitative analysis of dose-effect relationships: the combined effects of multiple drugs or enzyme inhibitors. *Advances in Enzyme Regulation,* 1984, vol. 22, 27-55
- **T.-C. CHOU.** Theoretical Basis, Experimental Design, and Computerized Simulation of Synergism and Antagonism in Drug Combination Studies. *Pharmacological Reviews,* 2006, vol. 58.3, 621-681
- **V. CHU et al.** Pharmacological Characterization of a Novel Factor Xa Inhibitor, FXV673. *Thrombosis Research,* 2001, vol. 103.4, 309-324
- **K. GUERTIN ; Y.-M. CHOI.** The Discovery of the Factor Xa Inhibitor Otamixaban: From Lead Identification to Clinical Development. *Current Medicinal Chemistry,* 2007, vol. 14.23, 2471-2481
- **A. PACCALY et al.** Pharmacokinetics of Otamixaban, a Direct Factor Xa Inhibitor, in Healthy Male Subjects: Pharmacokinetic Model Development for Phase 2/3 Simulation of Exposure. *The Journal of Clinical Pharmacology,* 2006, vol. 46.1, 37-44
- **L. HEDSTROM.** Serine Protease Mechanism and Specificity. *Chemical Reviews,* 2002, vol. 102.12, 4501-4524
- **M. Y. ABOU-ISMAIL ; A. DIAMOND ; S. KAPOOR ; Y. ARAFAH ; L. NAYAK.** The hypercoagulable state in COVID-19: Incidence, pathophysiology, and management. *Thrombosis Research,* 2020, vol. 194, 101-115
- **A. PACCALY et al.** Pharmacodynamic markers in the early clinical assessment of otamixaban, a direct factor Xa inhibitor. *Thrombosis and Haemostasis,* 2005, vol. 94.12, 1156-1163
- **C. BRINKMANN et al.** The Glycoprotein of Vesicular Stomatitis Virus Promotes Release of Virus-like Particles from Tetherin-Positive Cells. *PLoS One,* 2017, vol. 12.12, e0189073
- **H. KLEINE-WEBER et al.** Mutations in the Spike Protein of Middle East Respiratory Syndrome Coronavirus Transmitted in Korea Increase Resistance to Antibody-Mediated Neutralization. *J Virol,* 2019, vol. 93.2
- **M. BERGER RENTSCH ; G. ZIMMER.** A Vesicular Stomatitis Virus Replicon-Based Bioassay for the Rapid and Sensitive Determination of Multi-Species Type I Interferon. *PLoS One,* 2011, vol. 6.10, e25858
- **V. NEUHAUS et al.** Assessment of the Cytotoxic and Immunomodulatory Effects of Substances in Human Precision-Cut Lung Slices. *J Vis Exp,* 2018, vol. 135
- **T. STERLING ; J. J. IRWIN.** ZINC 15 - Ligand Discovery for Everyone. *Journal of Chemical Information and Modeling,* 2015, vol. 55.11, 2324-2337
- **G. M. MORRIS et al.** AutoDock4 and AutoDockTools4: Automated docking with selective receptor flexibility. *Journal of Computational Chemistry,* 2009, vol. 30.16, 2785-2791
- **D. R. KOES ; M. P. BAUMGARTNER ; C. J. CAMACHO.** Lessons Learned in Empirical Scoring with smina from the CSAR 2011 Benchmarking Exercise. *Journal of Chemical Information and Modeling,* 2013, vol. 53.8, 1893-1904
- **O. TROTT ; A. J. OLSON.** AutoDock Vina: Improving the speed and accuracy of docking with a new scoring function, efficient optimization, and multithreading. *Journal of Computational Chemistry,* 2009
- **R. QUIROGA ; M. A. VILLARREAL.** Vinardo: A Scoring Function Based on Autodock Vina Improves Scoring, Docking, and Virtual Screening. *PLOS ONE,* 2016, vol. 11.5, e0155183
- **P. EASTMAN et al.** OpenMM 7: Rapid Development of High Performance Algorithms for Molecular Dynamics. *PLOS Comput. Biol.,* 2017, vol. 13.7, e1005659
- **J. A. MAIER et al.** ff14SB: Improving the Accuracy of Protein Side Chain and Backbone Parameters from ff99SB. *J. Chem. Theory Comput.,* 2015, vol. 11.8, 3696-3713
- **Y. QIU et al.** Development and Benchmarking of Open Force Field v1.0.0. *Parsley Small Molecule Force Field,* 2020
- **W. L. JORGENSEN ; J. CHANDRASEKHAR ; J. D. MADURA ; R. W. IMPEY ; M. L. KLEIN.** Comparison of Simple Potential Functions for Simulating Liquid Water. *J. Chem. Phys.,* 1983, vol. 79.2, 926-935

- **C. W. HOPKINS ; S. LE GRAND ; R. C. WALKER ; A. E. ROITBERG.** Long-Time-Step Molecular Dynamics through Hydrogen Mass Repartitioning. *J. Chem. Theory Comput.,* 2015, vol. 11.4, 1864-1874
- **F. RAO ; M. SPICHTY.** Thermodynamics and Kinetics of Large-Time-Step Molecular Dynamics. *J. Comput. Chem.,* 2012, vol. 33.5, 475-483
- **W. HUMPHREY ; A. DALKE ; K. SCHULTEN.** VMD: Visual Molecular Dynamics. *J. Mol. Graph.,* 1996, vol. 14.1, 33-38
- **M. K. SCHERER et al.** PyEMMA 2: A Software Package for Estimation, Validation, and Analysis of Markov Models. *J. Chem. Theory Comput.,* 2015, vol. 11.11, 5525-5542
- **H. WU ; F. NOÉ.** Variational Approach for Learning Markov Processes from Time Series Data. *J Nonlinear Sci,* 2019
- **F. NOÉ ; H. WU ; J.-H. PRINZ ; N. PLATTNER.** Projected and Hidden Markov Models for Calculating Kinetics and Metastable States of Complex Molecules. *J. Chem. Phys.,* 2013, vol. 139.18, 184114
- **L. R. RABINER.** A Tutorial on Hidden Markov Models and Selected Applications in Speech Recognition. *Proc. IEEE,* 1989, vol. 77.2, 257-286
- **P. VIRTANEN et al.** SciPy 1.0: Fundamental Algorithms for Scientific Computing in Python. *Nat. Methods,* 2020, vol. 17, 261-272